# EUROPEAN PATENT APPLICATION

(11) **EP 4 386 370 A1**
(43) Date of publication of application: **19.06.2024**
(21) Application number: 22855119.8
(22) Date of filing: 01.07.2022
(51) Int. Cl.: G01N 27/62, G01N 1/28, G01N 1/30, C12Q 1/04

(54) **ANALYSIS METHOD FOR BACTERIUM IDENTIFICATION AND ANTIBIOTIC SENSITIVITY TESTING IN BIOLOGICAL SAMPLE**

(30) Priority: 12.08.2021 CN 202110923179
(71) Applicant: University of Science and Technology of China, Anhui 230026 (CN)
(72) Inventor: HUANG, Guangming, Hefei Anhui 230026 (CN); ZHAN, Liujuan, Hefei Anhui 230026 (CN); HOU, Zhuanghao, Hefei Anhui 230026 (CN)
(74) Representative: Nederlandsch Octrooibureau
(86) International application number: PCT/CN2022/103304
(87) International publication number: WO 2023/016135

(57) **Abstract**

A method for identifying a bacterium in a biological sample, and a method for testing antibiotic susceptibility of a bacterium in a biological sample, comprising: step (1), centrifugally separating the bacterium from the biological sample; step (2), staining the bacterium obtained in step (1); step (3), fixing the stained bacterium in step (2) by electrostatic adsorption; step (4), injecting an extraction solution into a capillary tip, and then extracting metabolites of the single living bacterium by in-situ extraction; step (5), inserting an electrode into the capillary obtained in step (4), applying a voltage, and performing mass spectrometry. The method does not depend on incubation, and the identification and the antibiotic susceptibility testing of a bacterium in a biological sample of an early bacterial infection can be realized, and the method has the advantages of being rapid, having high sensitivity, capable of perfroming a non-targeting analysis, etc., and has good application prospects.

## Description

### FIELD OF THE INVENTION

The present disclosure relates to the biological field. Specifically, the present disclosure relates to analytical methods for bacterium identification and antibiotic susceptibility testing in biological samples.

### BACKGROUND OF THE INVENTION

The detection or identification of bacteria is required in many different fields, and rapid and accurate identification of bacteria is crucial in fields such as medicine and biology. Some biological samples have low bacterial contents in the early stage of bacterial infection. For example, in the early stage of blood infection, there are only 1-1000 bacteria per milliliter of blood. With the overuse and abuse of antibiotics, more and more bacteria are mutating, which puts forward more stringent requirements for rapid and accurate bacterial detection.

Culture method is currently the most commonly used method for detecting bacteria in biological samples. The density of bacteria increases through long-term culture, so that bacteria can be identified and subsequently analyzed. However, the culture method takes a long time, cannot obtain test results in time, has a large hysteresis, and cannot be used to detect some viable nonculturable bacteria. Therefore, in order to solve these problems, researchers have developed many culture-free methods to achieve rapid detection of bacteria in biological samples. For example, the fluorescence-based method can detect individual bacterium in a biological sample with high sensitivity, but it requires information about known bacteria to target them, so the fluorescence-based method cannot accurately determine unknown bacteria.

Mass spectrometry is a non-targeting analytical method that enables simultaneous analysis of multiple metabolites based on the differences in mass-to-charge ratios of various analytes. Mass spectrometry does not require labels and does not require prior knowledge of analyte information, and thus it can quickly identify various unknown components and is widely used in the analysis of unknown biological samples. However, individual bacterium is extremely small and has low metabolite content, which brings huge challenges to the analysis of individual bacterium by mass spectrometry. At present, mass spectrometry analysis is still based on culture in terms of bacterial detection, wherein a single bacterium is incubated into a colony and then mass spectrometry analysis is performed. The sensitivity of the mass spectrometry analytical method cannot yet reach the sensitivity of a single bacterium. Therefore, mass spectrometry analysis of a single bacterium requires the development of a highly sensitive mass spectrometry analytical method.

### SUMMARY OF THE INVENTION

The purpose of the present disclosure includes providing a method for rapid and accurate identification of bacteria in a biological sample directly, without incubation. In view of the shortcomings of existing detection methods, the present disclosure provides a mass spectrometry analytical method for a single living bacterium, wherein, metabolite of a single living bacterium in a biological sample is extracted by in-situ extraction and then subjected to mass spectrometry detection to achieve identification of a single living bacterium in a biological sample and antibiotic susceptibility testing; the process of biological sample treatment, single living bacterium sampling and mass spectrometry detection takes less than 1 hour, which meets the demands for rapid and accurate bacterial analysis in actual biological samples.

The present disclosure provides the following:
The present disclosure provides a method for identifying a bacterium in a biological sample, comprising the steps of staining and fixing the bacterium in the biological sample, and extracting metabolite of the fixed single living bacterium and then performing mass spectrometry detection.

In some embodiments, the metabolite(s) of the fixed single living bacterium is extracted via a capillary tube.

In some embodiments, the capillary tube is pre-pulled before use.

In some embodiments, the fixation is performed by electrostatic interaction or covalent binding, and the slide for fixing includes, but is not limited to, a polylysine slide, a polyethylenimine slide, a gelatin slide, an N-(2-aminoethyl)-3-aminopropyl trimethoxysilane slide, and a 3-aminopropyltriethoxysilane slide.

In some embodiments, the slide for fixing is selected from a polylysine slide, a polyethylenimine slide, a gelatin slide, an N-(2-aminoethyl)-3-aminopropyl trimethoxysilane slide, and a 3-aminopropyltriethoxysilane slide.

In some embodiments, before the staining of the bacterium, a step of precipitating a large particle interfering substance from the biological sample by low-speed centrifugation is performed.

In some embodiments, the method for identifying a bacterium in a biological sample includes the following steps:
Step (1): precipitating a large particle interfering substance from the biological sample via a low-speed centrifugation, retaining the supernatant containing the bacterium to obtain the bacterium of the biological sample;
Step (2): staining the bacterium in the supernatant obtained in Step (1) to locate the bacterium, and performing a high-speed centrifugation to wash away the staining agent;
Step (3): droping the solution containing the stained bacterium obtained in Step (2) onto a slide, capturing and fixing the bacterium by electrostatic adsorption or covalent binding;
Step (4): injecting an extraction solution into a capillary, and performing under a microscope an in-situ extraction of metabolites from the fixed single living bacterium obtained in Step (3);
Step (5): inserting an electrode into the capillary of Step (4), applying a voltage for electrospray, and analyzing the extracted bacterial metabolite by mass spectrometry.

In some embodiments, the biological sample includes, but is not limited to, human whole blood, rabbit whole blood, urine samples, saliva, cerebrospinal fluid, alveolar lavage fluid, abscesses, cell solution infected by bacteria, and tissue suspensions infected by bacteria.

In some embodiments, the biological sample is selected from human whole blood, rabbit whole blood, urine samples, saliva, cerebrospinal fluid, alveolar lavage fluid, abscesses, cell solution infected by bacteria, and tissue suspensions infected by bacteria.

In some embodiments, the bacterium includes, but is not limited to, Escherichia coli, Staphylococcus aureus, Pseudomonas aeruginosa, and Acinetobacter baumannii.

In some embodiments, the bacterium is selected from Escherichia coli, Staphylococcus aureus, Pseudomonas aeruginosa, and Acinetobacter baumannii.

In some embodiments, the rotation speed of low-speed centrifugation is 2000rpm-4000rpm, and the centrifugation time is 5min-10min.

In some embodiments, the rotation speed of high-speed centrifugation is 10000rpm-14000rpm, and the centrifugation time is 5min-10min.

In some embodiments, the staining agent includes, but is not limited to, Acridine Orange, Fluorescein Diacetate, Syto 9, Syto 64, hoechst 33258, and DAPI.

In some embodiments, the staining agent is selected from Acridine Orange, Fluorescein Diacetate, Syto 9, Syto 64, hoechst 33258, and DAPI.

In some embodiments, the staining is performed for 10 min-30 min with a concentration of 1 µM-10 µM for the staining agent, and the staining is protected from light.

In some embodiments, the interaction time between the solution containing the bacterium and the slide is 10-30 minutes.

In some embodiments, the opening size of the capillary tip is less than 2µm, preferably 500nm-1µm;

In some embodiments, the extraction solution is one or more of, but not limited to, water, methanol, ethanol, acetonitrile, acetone, chloroform, formic acid, acetic acid, and trifluoroacetic acid.

In some embodiments, the extraction solution is one or more selected from the group consisting of water, methanol, ethanol, acetonitrile, acetone, chloroform, formic acid, acetic acid and trifluoroacetic acid.

In some embodiments, the method further includes a step of establishing a bacterium comparison database.

In some embodiments, the establishment of a bacterium comparison database includes the following steps:
(a) performing a mass spectrometry analysis of single living bacterium metabolites for one or more reference species;
(b) conducting a dimensionality reduction and visual analysis on the reference living bacterium of one or more species based on the metabolite analyzed in step (a), and classifying different bacteria based on the metabolite of the single living bacteriun obtained in step (a) to establish a comparison database for the bacterial identification.

On the other hand, the present disclosure provides a method for testing the antibiotic susceptibility of a bacterium in a biological sample, comprising the above method, and optionally also includes the step of incubating the bacterium in the biological sample with an antibiotic.

In some embodiments, the method for testing the antibiotic susceptibility of a bacterium in a biological sample includes the following steps:
Step (1): precipitating a large particle interfering substance from the biological sample through low-speed centrifugation, and the supernatant containing the bacterium is retained to obtain the bacterium in the biological sample;
Step (2): adding the supernatant containing the bacterium into solutions containing different concentrations of the antibiotic and incubating them;
Step (3): centrifuging the incubated solutions containing the bacterium, discarding the supernatants, and resuspending the precipitation in deionized water;
Step (4): staining the bacterium obtained in Step (3) to locate the bacterium, and centrifuging at high speed after staining to wash away the staining agent;
Step (5): droping the solution containing the stained bacterium obtained in Step (4) onto a slide, capturing and fixing the bacterium sample by electrostatic adsorption or covalent binding;
Step (6): injecting an extraction solution into a capillary, and performing under a microscope an in-situ extraction of metabolites from the fixed single living bacterium obtained in Step (5);
Step (7): inserting an electrode into the capillary of Step (6), applying a voltage for electrospray, and analyzing the extracted bacterial metabolite by mass spectrometry.

### DEFINITIONS

T-SNE (t-distributed stochastic neighbor embedding) is a machine learning algorithm for dimensionality reduction, which was proposed by Laurens van der Maaten and Geoffrey Hinton in 2008. In addition, T-SNE is a nonlinear dimensionality reduction algorithm that is very suitable for reducing high-dimensional data to 2 or 3 dimensions for visualization. Through the programmed MATLAB running script, t-SNE analysis can be performed on a lot of high-dimensional bacterial mass spectrometry data; bacterial mass spectrometry data can be processed in batches, and significance indicators can be automatically exported, and thus differences in bacterial mass spectrometry data can be distinguished and effective typing can be performed.

### The present disclosure has the following advantages:

(1) The method of the present disclosure can be performed quickly without incubation. It can analyze a single living bacterium in a biological sample infected by bacteria, and the process of biological sample treatment, single living bacteria sampling and mass spectrometry detection takes less than 1 hour;
(2) The method of the present disclosure has a high sensitivity and can perform mass spectrometry analysis of metabolites of a single living bacterium;
(3) The method of the present disclosure is a non-targeting detection, which can accurately analyze unknown bacteria, identify different bacteria based on differences of metabolites, and analyze the metabolic response of bacteria after antibiotic stimulation.

### BRIEF DESCRIPTION OF THE DRAWINGS

Figure 1 shows a flow chart of mass spectrometry analysis of a single living bacterium;
Figure 2 shows a flow chart of sorting and capturing and fixing bacteria in a whole blood according to an embodiment of the present disclosure;
Figure 3 shows a bright field image and a fluorescence image after capturing and fixing bacteria according to an embodiment of the present disclosure;
Figure 4 shows an in-situ extraction of a single living bacterium according to an embodiment of the present disclosure;
Figure 5 shows the spectrum of metabolites of single bacterium according to an embodiment of the present disclosure;
Figure 6 shows a t-SNE diagram of Escherichia coli, Acinetobacter baumannii, and Staphylococcus aureus according to an embodiment of the present disclosure;
Figure 7 is a neural network test diagram showing the identification accuracy of Escherichia coli, Acinetobacter baumannii, and Staphylococcus aureus according to an embodiment of the present disclosure;
Figure 8 shows a t-SNE diagram of bacterial species identification according to one embodiment of the present disclosure;
Figure 9 shows a t-SNE diagram of bacterial species identification according to one embodiment of the present disclosure;
Figure 10 shows a t-SNE diagram of bacterial species identification according to one embodiment of the present disclosure;
Figure 11 shows a t-SNE diagram of bacterial species identification according to one embodiment of the present disclosure;
Figure 12 shows a response graph of changes in metabolites of single Escherichia coli after stimulation with different antibiotics according to an embodiment of the present disclosure;
Figure 13 shows a response graph of changes in metabolites of single Escherichia coli after stimulation with different concentrations of kanamycin according to an embodiment of the present disclosure.

### DETAILED DESCRIPTION OF THE INVENTION

In order to make the purposes, technical solutions, and advantages of the present disclosure more clear, the present disclosure will be further described in details below with reference to specific Examples and Figures.

### Example 1 Detection and identification of a single living bacterium in a whole blood, comprising the following steps:

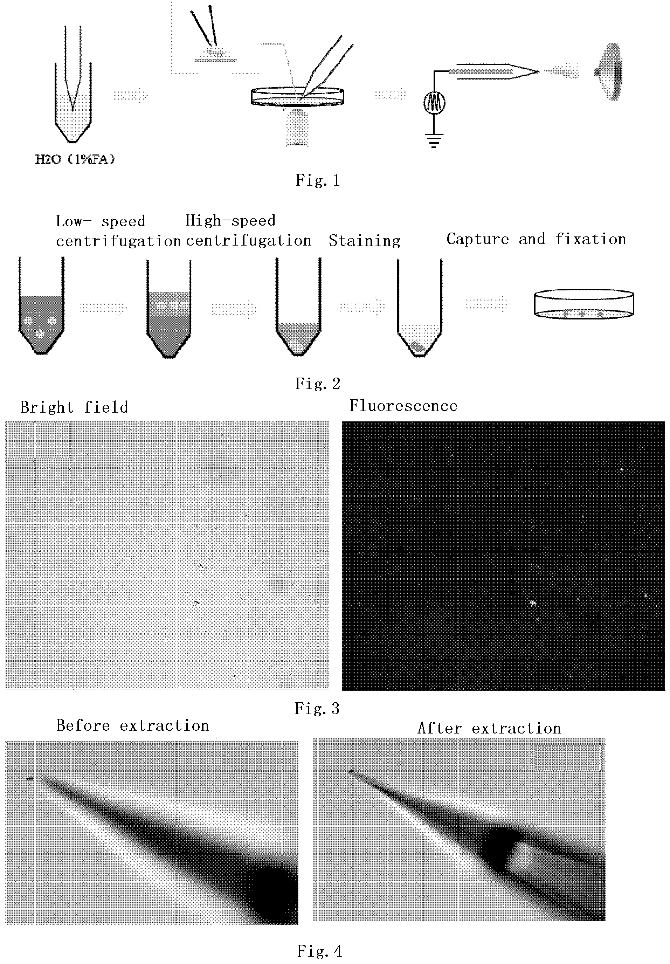

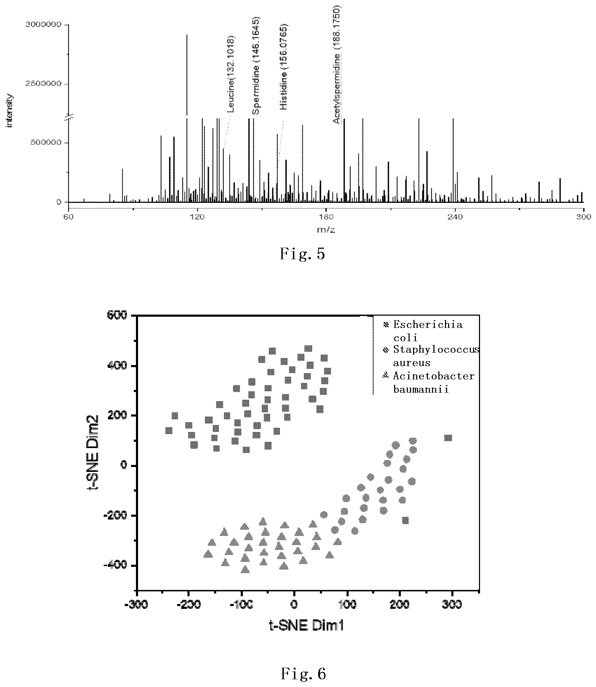

(1) Capture and localization of bacteria in the whole blood:
1 mL of whole blood infected by bacteria was taken and placed in a centrifuge tube, centrifuged at 3000 rpm for 8 min; the blood cell pellet was discarded; 500 µL of supernatant containing the bacterium was centrifuged at 10000 rpm for 8 min, and the supernatant was discarded; the pellet was resuspended in 100 µL of deionized water; 10 µL of 100 µM Acridine Orange as a staining agent was added to perform staining for 15 minutes, the stained solution was centrifuged at 10,000 rpm for 8 minutes; the staining agent was washed away, and the pellet was resuspended in 100 µL of deionized water. The resuspended solution containing the bacterium was dropped onto a polylysine slide and leave for 30 minutes to allow the bacterium to be adsorbed and fixed on the polylysine slide, and then the polylysine slide was rinsed with deionized water and dried at room temperature; the location of bacteria was observed under a fluorescence microscope as shown in Figure 3.
(2) Capillary preparation:
A capillary with a tip opening size of 1 µm was drawn by a P-2000 laser-based micropipette puller;
(3) In-situ extraction of metabolites in a single living bacterium:
The capillary drawn in step (2) was inserted into a deionized water (containing 1% formic acid) solution, and then taken out and fixed on a micro-operating platform, wherein the capillary was positioned under a microscope to contact the bacterium, and the solution in the capillary was pushed out via a syringe pump to extract the metabolites of bacteria for 1 second and then the solution was sucked back. The in-situ extraction of a single living bacterium was shown in Figure 4;
(4) Mass spectrometry analysis:
The capillary after extraction was placed in the front of the mass spectrometer inlet; a voltage was applied; and the mass spectrum was collected as shown in Figure 5;
(5) Establishment of a bacterium comparison database:
1 µL of solution containing the bacterium with a concentration of 10⁵ cells/mL was dropped onto the surface of the polylysine slide, and then leave for for 30 minutes to allow the bacterium to be adsorbed on the surface of the polylysine slide; the slide was washed, and dried at room temperature; metabolites of a single living bacterium were extracted via deionized water (containing 1% formic acid) and analyzed by mass spectrometry. Table 1 showed the set of metabolites detected in three bacteria: Escherichia coli, Acinetobacter baumannii, and Staphylococcus aureus. T-distributed stochastic neighbor embedding (t-SNE) analysis was performed on the three bacteria based on the measured metabolites. Figure 6 is the t-distributed stochastic neighbor embedding (t-SNE) analysis for the high-dimensional data of the metabolite fingerprints of three bacteria: Escherichia coli, Acinetobacter baumannii, and Staphylococcus aureus. The measured metabolites of single bacterium can be used to classify different bacteria and establish a database for the identification of unknown bacteria.

**Table 1: List of metabolites detected in Escherichia coli, Acinetobacter baumannii, and Staphylococcus aureus.**

| Metabolites | molecular formula | Ion type | theoretical m/z | measured m/z | Error (ppm) |
|---|---|---|---|---|---|
| Methylalanine | C₄H₉NO₂ | M+H | 104.0706 | 104.0706 | 0 |
| Creatinine | C₄H₇N₃O | M+H | 114.0660 | 114.0662 | 1 |
| Proline | C₅H₉NO₂ | M+H | 116.0703 | 116.0706 | 3 |
| Valine | C₅H₁₁NO₂ | M+H | 118.0859 | 118.0863 | 3 |
| Agmatine | C₅H₁₄N₄ | M+H | 131.1287 | 131.1291 | 3 |
| Creatine | C₄H₉N₃O₂ | M+H | 132.0763 | 132.0768 | 4 |
| Leucine | C₆H₁₃NO₂ | M+H | 132.1012 | 132.1019 | 5 |
| Ornithine | C₅H₁₂N₂O₂ | M+H | 133.0966 | 133.0972 | 4 |
| Urocanic acid | C₆H₆N₂O₂ | M+H | 139.0496 | 139.0502 | 4 |
| Spermidine | C₇H₁₉N₃ | M+H | 146.1646 | 146.1652 | 4 |
| Histidine | C₆H₉N₃O₂ | M+H | 156.0761 | 156.0768 | 4 |
| Arginine | C₆H₁₄N₄O₂ | M+H | 175.1182 | 175.1190 | 4 |
| Argininic acid | C₆H₁₃N₃O₃ | M+H | 176.1022 | 176.1030 | 4 |
| Acetylspermidine | C₉H₂₁N₃O | M+H | 188.1747 | 188.1757 | 6 |
| Alanylthreonine | C₇H₁₄N₂O₄ | M+H | 191.1035 | 191.1026 | 4 |

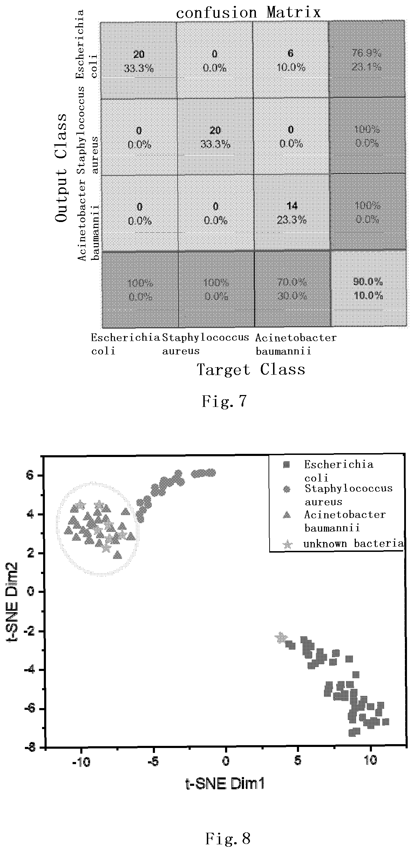

(6) Test of bacterial species identification accuracy:
In order to verify the accuracy of the bacterial metabolite database established in step (5) for identifying a bacterial species, the metabolites detected in Escherichia coli, Acinetobacter baumannii and Staphylococcus aureus were used as a test set to conduct a neural network test. Figure 7 was the neural network test diagram for the accuracy of identification of three types of bacteria, wherein the bacterial metabolite database established in step (5) had an accuracy of 90% for the identification of bacteria.
(7) Determination of species for bacteria
The bacterial metabolite fingerprints obtained in step (4) were input into the bacterial database established in step (5), and the bacterium were classified into different types based on the differences in detected metabolites. The identification of bacterial species detected in step (4) was shown in Figure 8. The detected spots fell into the range of Acinetobacter baumannii in the database, indicating that the bacterium detected in step (4) were Acinetobacter baumannii.

### Example 2 The detection and identification of a single living bacterium in cells infected by bacteria, comprising the following steps:

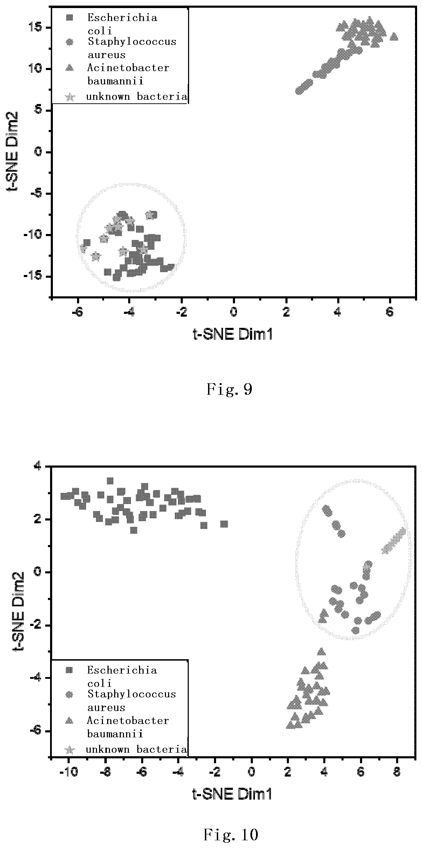

(1) Isolation of bacteria in cells:
   100 µL of 10% saponin was added into 1 mL of RAW264.7 cell suspension infected with bacteria; the cells were lysed at 37°C for 5 min, centrifuged at 3000 rpm for 8 min; the pellet was discarded, and the supernatant containing the bacterium was retained.
(2) Capture and localization of bacteria:
   10 µL of 100 µM Acridine Orange as a staining agent was added into the solution containing the bacterium obtained in step (1) to perform staining for 15 min. The stained solution was centrifuged at 10,000 rpm for 8 min. The staining agent was washed away, and the pellet was resuspended in 100 µL of deionized water. The resuspended solution containing the bacterium was dropped onto a polylysine slide and leave for 30 minutes to allow the bacterium to be adsorbed and fixed on the polylysine slide, and then the polylysine slide was rinsed with deionized water and dried at room temperature; the location of bacteria was observed under a fluorescence microscope.
(3) Capillary preparation:
   A capillary with a tip diameter opening size of 1 µm was drawn by a P-2000 laser-based micropipette puller;
(4) In-situ extraction of metabolites of a single living bacterium:
   The capillary drawn in step (3) was inserted into a deionized water (containing 1% formic acid) solution, and then taken out and fixed on a micro-operating platform, wherein the capillary was positioned under a microscope to contact the bacterium, and the solution in the capillary was pushed out via a syringe pump to extract the metabolites of a single living bacterium for 1 second and then the solution was sucked back.
(5) Mass spectrometry analysis:
   The capillary after extraction was placed at the front of the mass spectrometer inlet; a voltage was applied; and the mass spectrum of the extract was collected.
(6) Establishment of a bacterium comparison database:
   1 µL of solution containing the bacterium with a concentration of 10⁵ cells/mL was dropped onto the surface of the polylysine slide, and then leave for 30 minutes to allow the bacterium to be adsorbed on the surface of the polylysine slide; the slide was washed, and dried at room temperature; metabolites of a single living bacterium were extracted via deionized water (containing 1% formic acid) and analyzed by mass spectrometry. Table 1 showed the set of metabolites detected in three bacteria: Escherichia coli, Acinetobacter baumannii, and Staphylococcus aureus. T-distributed stochastic neighbor embedding (t-SNE) analysis was performed on the three bacteria based on the measured metabolites. Figure 6 is the t-distributed stochastic neighbor embedding (t-SNE) analysis for the high-dimensional data of the metabolite fingerprints of three bacteria: Escherichia coli, Acinetobacter baumannii, and Staphylococcus aureus. The measured metabolites of single bacterium can be used to classify different bacteria and establish a database for the identification of unknown bacteria.
(7) Test of bacterial species identification accuracy:
   In order to verify the accuracy of the bacterial metabolite database established in step (6) for identifying a bacterial species, the metabolites detected in Escherichia coli, Acinetobacter baumannii and Staphylococcus aureus were used as a test set to conduct a neural network test. Figure 7 was the neural network test diagram for the accuracy of identification of three types of bacteria, wherein the bacterial metabolite database established in step (6) had an accuracy of 90% for the identification of bacteria.
(8) Determination of species for bacteria
   The bacterial metabolite fingerprints obtained in step (5) were input into the bacterial database established in step (6), and the bacterium were classified into different types based on the differences in detected metabolites. The identification of bacterial species detected in step (5) was shown in Figure 9. The detected spots fell into the range of Escherichia coli in the database, indicating that the bacterium detected in step (5) was Escherichia coli.

### Example 3 The detection and identification of a single living bacterium in tissues infected by bacteria, comprising the following steps:

(1) Isolation of bacteria in infected tissues:
   The infected tissue was wiped by a sterile cotton swab, which was then inserted into 1 mL of deionized water to elute the bacterium, and the eluate containing the bacterium was retained.
(2) Capture and localization of bacteria:
   10 µL of 1mM Acridine Orange as a staining agent was added into the solution containing the bacterium obtained in step (1) to perform staining for 15 min. The stained solution was centrifuged at 10,000 rpm for 8 min. The staining agent was washed away, and the pellet was resuspended in 100 µL of deionized water. The resuspended solution containing the bacterium was dropped onto a polylysine slide and leave for 30 minutes to allow the bacterium to be adsorbed and fixed on the polylysine slide, and then the polylysine slide was rinsed with deionized water and dried at room temperature; the location of bacteria was observed under a fluorescence microscope.
(3) Capillary preparation:
   A capillary with a tip diameter opening size of 1 µm was drawn by a P-2000 laser-based micropipette puller;
(4) In-situ extraction of metabolites of a single living bacterium:
   The capillary drawn in step (3) was inserted into a deionized water (containing 1% formic acid) solution, and then taken out and fixed on a micro-operating platform, wherein the capillary was positioned under a microscope to contact the bacterium, and the solution in the capillary was pushed out via a syringe pump to extract the metabolites of a single living bacterium for 1 second and then the solution was sucked back.
(5) Mass spectrometry analysis:
   The capillary after extraction was placed at the front of the mass spectrometer inlet; a voltage was applied; and the mass spectrum was collected.
(6) Establishment of a bacterium comparison database:
   1 µL of solution containing the bacterium with a concentration of 10⁵ cells/mL was dropped onto the surface of the polylysine slide, and then leave for 30 minutes to allow the bacterium to be adsorbed on the surface of the polylysine slide; the slide was washed, and dried at room temperature; metabolites of a single living bacterium were extracted via deionized water (containing 1% formic acid) and analyzed by mass spectrometry. Table 1 showed the set of metabolites detected in three bacteria: Escherichia coli, Acinetobacter baumannii, and Staphylococcus aureus. T-distributed stochastic neighbor embedding (t-SNE) analysis was performed on the three bacteria based on the measured metabolites. Figure 6 is the t-distributed stochastic neighbor embedding (t-SNE) analysis for the high-dimensional data of the metabolite fingerprints of three bacteria: Escherichia coli, Acinetobacter baumannii, and Staphylococcus aureus. The measured metabolites of a single bacterium can be used to classify different bacteria and establish a database for the identification of unknown bacteria.
(7) Test of bacterial species identification accuracy:
   In order to verify the accuracy of the bacterial metabolite database established in step (6) for identifying a bacterial species, the metabolites detected in Escherichia coli, Acinetobacter baumannii and Staphylococcus aureus were used as a test set to conduct a neural network test. Figure 7 was the neural network test diagram for the accuracy of identification of three types of bacteria, wherein the bacterial metabolite database established in step (6) had an accuracy of 90% for the identification of bacteria.
(8) Determination of species for bacteria
   The bacterial metabolite fingerprints obtained in step (5) were input into the bacterial database established in step (6), and the bacterium were classified into different types based on the differences in detected metabolites. The identification of bacterial species detected in step (5) was shown in Figure 10. The detected spots fell into the range of Staphylococcus aureus in the database, indicating that the bacterium detected in step (5) was Staphylococcus aureus.

### Example 4 The detection and identification of a single living bacterium in urine, comprising the following steps:

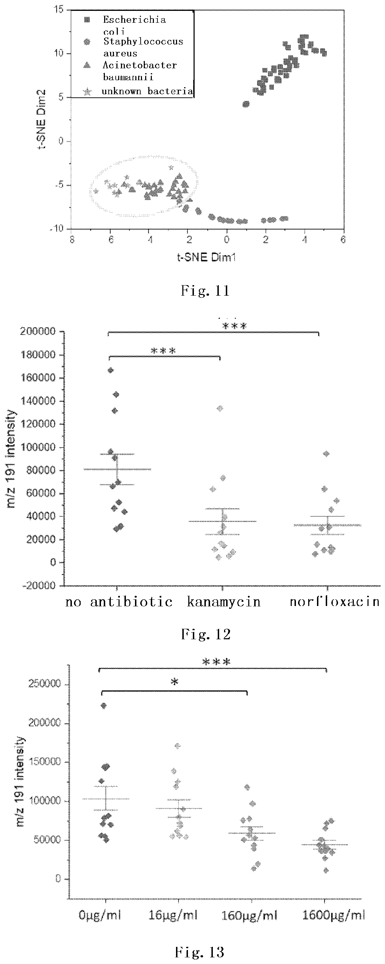

(1) Capture and localization of bacteria in urine:
   1 mL of urine from patients with urinary tract infections was taken and placed in a centrifuge tube, centrifuged at 3000 rpm for 8 min; the large particle pellet was discarded; 800 µL of supernatant containing the bacterium was centrifuged at 10000 rpm for 8 min, and the supernatant was discarded; the pellet was resuspended in 100 µL of deionized water; 10 µL of 100 µM Acridine Orange as a staining agent was added to perform staining for 15 minutes, the stained solution was centrifuged at 10,000 rpm for 8 minutes; the staining agent was washed away, and the pellet was resuspended in 100 µL of deionized water. The resuspended solution containing the bacterium was dropped onto a polylysine slide and leave for 30 minutes to allow the bacterium to be adsorbed and fixed on the polylysine slide, and then the polylysine slide was rinsed with deionized water and dried at room temperature; the location of bacteria was observed under a fluorescence microscope.
(2) Capillary preparation:
   A capillary with a tip diameter opening size of 1 µm was drawn by a P-2000 laser-based micropipette puller;
(3) In-situ extraction of metabolites of a single living bacterium:
   The capillary drawn in step (2) was inserted into a deionized water (containing 1% formic acid) solution, and then taken out and fixed on a micro-operating platform, wherein the capillary was positioned under a microscope to contact the bacterium, and the solution in the capillary was pushed out via a syringe pump to extract the metabolites of bacteria for 1 second and then the solution was sucked back;
(4) Mass spectrometry analysis:
   The capillary after extraction was placed at the front of the mass spectrometer inlet; a voltage was applied; and the mass spectrum was collected;
(5) Establishment of a bacterium comparison database:
   1 µL of solution containing the bacterium with a concentration of 10⁵ cells/mL was dropped onto the surface of the polylysine slide, and then leave for 30 minutes to allow the bacterium to be adsorbed on the surface of the polylysine slide; the slide was washed, and dried at room temperature; metabolites of a single living bacterium were extracted via deionized water (containing 1% formic acid) and analyzed by mass spectrometry. Table 1 showed the set of metabolites detected in three bacteria: Escherichia coli, Acinetobacter baumannii, and Staphylococcus aureus. T-distributed stochastic neighbor embedding (t-SNE) analysis was performed on the three bacteria based on the measured metabolites. Figure 6 is the t-distributed stochastic neighbor embedding (t-SNE) analysis for the high-dimensional data of the metabolite fingerprints of three bacteria: Escherichia coli, Acinetobacter baumannii, and Staphylococcus aureus. The measured metabolites of single bacterium can be used to classify different bacteria and establish a database for the identification of unknown bacteria.
(6) Test of bacterial species identification accuracy:
   In order to verify the accuracy of the bacterial metabolite database established in step (5) for identifying a bacterial species, the metabolites detected in Escherichia coli, Acinetobacter baumannii and Staphylococcus aureus were used as a test set to conduct a neural network test. Figure 7 was the neural network test diagram for the accuracy of identification of three types of bacteria, wherein the bacterial metabolite database established in step (5) had an accuracy of 90% for the identification of bacteria.
(7) Determination of species for bacteria
   The bacterial metabolite fingerprints obtained in step (4) were input into the bacterial database established in step (5), and the bacterial species was determined based on the differences in detected metabolites. The identification of bacterial species detected in step (4) was shown in Figure 11. The detected spots fell into the range of Acinetobacter baumannii in the database, indicating that the bacterium detected in step (4) was Acinetobacter baumannii.

### Example 5 Antibiotic susceptibility test of a single living bacterium in the whole blood, comprising the following steps:

(1) Incubation of bacteria with different antibiotics:
   1 mL of whole blood was taken and placed in a centrifuge tube, centrifuged at 3000 rpm for 8 min; the blood cell pellet was discarded; 10 µL of 8 mg/mL kanamycin and 10 µL of 2 mg/mL norfloxacin were respectively added into 500 µL of the supernatant containing the bacterium, and incubated with shaking at 37°C. After 30 min of incubation, the supernatant was centrifuged at 10,000 rpm for 8 min; the supernatant was discarded, and the pellet was resuspended in 100 µL of deionized water; the solution containing the bacterium not having been incubated with the antibiotic was used as a reference group, and the same operations as above were performed.
(2) Incubation of bacteria with kanamycin at different concentrations;
   1 mL of whole blood was taken and placed in a centrifuge tube, centrifuged at 3000 rpm for 8 min; the blood cell pellet was discarded; 10 µL of 0.8 mg/mL, 8 mg/mL, and 80 mg/mL kanamycin were respectively added into 500 µL of the supernatant containing the bacterium, and incubated with shaking at 37°C. After 30 min of incubation, the supernatant was centrifuged at 10,000 rpm for 8 min; the supernatant was discarded, and the pellet was resuspended in 100 µL of deionized water; the solution containing the bacterium not having been incubated with the antibiotic was used as a reference group, and the same operations as above were performed.
(3) Capture and localization of bacteria:
   10 µL of 100µM Acridine Orange as a staining agent was added into the solution containing the bacterium obtained in step (1) and step (2) to perform staining for 15 min. The stained solution was centrifuged at 10,000 rpm for 8 min. The staining agent was washed away, and the pellet was resuspended in 100 µL of deionized water. The resuspended solution containing the bacterium was dropped onto a polylysine slide and leave for 30 minutes to allow the bacterium to be adsorbed and fixed on the polylysine slide, and then the polylysine slide was rinsed with deionized water and dried at room temperature; the location of bacteria was observed under a fluorescence microscope.
(4) Capillary preparation:
   A capillary with a tip diameter opening size of 1 µm was drawn by a P-2000 laser-based micropipette puller;
(5) In-situ extraction of metabolites of a single living bacterium:
   The capillary drawn in step (3) was inserted into a deionized water (containing 1% formic acid) solution, and then taken out and fixed on a micro-operating platform, wherein the capillary was positioned under a microscope to contact the bacterium, and the solution in the capillary was pushed out via a syringe pump to extract the metabolites of the bacterium for 1 second and then the solution was sucked back.
(6) Mass spectrometry analysis:
   The capillary after extraction was placed at the front of the mass spectrometer inlet; a voltage was applied; and the mass spectrum was collected;
(7) Determination of antibiotic susceptibility:
   The signal intensity changes of bacterial metabolites after incubation with different antibiotics were compared. When the bacterium were stimulated by a sensitive antibiotic, the signal intensities of metabolites detected in a single bacterium, such as creatinine, proline, creatine, leucine, spermidine, histidine, arginine, acetyl spermidine, and alanyl-threonine, would all change significantly. Taking the metabolite m/z 191 alanyl-threonine in a single bacterium as an example, Figure 12 showed the response chart of the change in the metabolite alanyl-threonine in a single bacterium after stimulation of E. coli with different antibiotics. After incubation with the sensitive antibiotics kanamycin and norfloxacin, the alanyl-threonine signal was significantly reduced, while incubation without antibiotics did not lead to significant changes in the alanylthreonine signal. Figure 13 showed the response chart of the change of alanyl-threonine in a single E. coli after stimulation with different concentrations of kanamycin. When the concentration of kanamycin reached a minimal inhibitory concentration (MIC), the alanylthreonine signal of a single E. coli decreased significantly, showing that the response of metabolite in single bacterium could be monitored by mass spectrometry of a living single bacterium, and the antibiotic susceptibility of bacterium can be determined based on changes in the detected metabolite signal intensity.

The specific Examples described above further describe the purposes, technical solutions and beneficial effects of the present disclosure in details, but they should not be construed as limiting the scope of the disclosure. It should be noted that any modifications, equivalent replacements and improvements made by those skilled in the art within the spirit and scope of the disclosure should be included within the protection scope of the present disclosure.

## Claims

1. A method for identifying a bacterium in a biological sample, comprising: staining and fixing the bacterium in the biological sample, extracting metabolites of the fixed single living bacterium, and then performing mass spectrometry detection; preferably, injecting an extraction solution into a capillary, positioning the capillary under a microscope to contact the single living bacterium, and extracting the metabolite from the single living bacterium.

2. The method for identifying a bacterium in a biological sample of claim 1, wherein the fixing is performed by electrostatic interaction or covalent binding, and a slide for the fixing is selected from a polylysine slide, a polyethylenimine slide, a gelatin slide, a N-(2-aminoethyl)-3-aminopropyl trimethoxysilane slide, and a 3-aminopropyltriethoxysilane slide; optionally, the interaction time between the bacterium and the slide is 10-30 minutes.

3. The method for identifying a bacterium in a biological sample of claim 1 or claim 2, wherein, before staining the bacterium, performing a step of precipitating a large particle interfering substance from the biological sample via a low-speed centrifugation.

4. The method for identifying a bacterium in a biological sample of any one of claims 1-3, comprising:
Step (1): precipitating a large particle interfering substance from the biological sample via a low-speed centrifugation (preferably the speed of the low-speed centrifugation being 2000rpm-4000rpm, and the centrifugation time being 5min-10min), retaining a supernatant containing the bacterium to obtain the bacterium of the biological sample;
Step (2): staining the bacterium in the supernatant obtained in Step (1) to locate the bacterium, and performing a high-speed centrifugation (preferably the speed of the high-speed centrifugation being 10000rpm-14000rpm, and the centrifugation time being 5min-10min) to wash away the staining agent;
Step (3): droping the solution containing the stained bacterium obtained in Step (2) onto a slide, capturing and fixing the bacterium by electrostatic adsorption or covalent binding;
Step (4): injecting an extraction solution into a capillary (for example, with an opening size of less than 2 µm, preferably 500 nm-1 µm), and performing an in-situ extraction of metabolites from the fixed single living bacterium obtained in Step (3) under a microscope;
Step (5): inserting an electrode into the capillary of Step (4), applying a voltage for electrospray, and analyzing the extracted bacterial metabolite by mass spectrometry.

5. The method for identifying a bacterium in a biological sample of any one of claims 1-4, wherein the biological sample is selected from a human whole blood, a rabbit whole blood, a urine sample, saliva, cerebrospinal fluid, alveolar lavage fluid, an abscess, a cell solution infected by the bacterium, and a tissue suspension infected by the bacterium; optionally, the bacterium is selected from Escherichia coli, Staphylococcus aureus, Pseudomonas aeruginosa, and Acinetobacter baumannii.

6. The method for identifying a bacterium in a biological sample of any one of claims 1-5, wherein the staining agent is selected from Acridine Orange, Fluorescein Diacetate, Syto 9, Syto 64, hoechst 33258 and DAPI; optionally, the staining is performed for 10 min-30 min at a staining agent concentration of 1 µM-10 µM, and the staining is protected from light.

7. The method for identifying a bacterium in a biological sample of claim 4, wherein the extraction solution is one or more selected from the group consisting of water, methanol, ethanol, acetonitrile, acetone, chloroform, formic acid, acetic acid and trifluoroacetic acid.

8. The method for identifying a bacterium in a biological sample of any one of claims 1-7, further comprising establishing a bacterium comparison database.

9. The method for identifying a bacterium in a biological sample of claim 8, wherein establishing the bacterium comparison database comprises the following steps:
(a) performing a mass spectrometry analysis of single living bacterium metabolites for one or more reference species;
(b) conducting a dimensionality reduction and visual analysis on the reference living bacterium of one or more species based on the metabolite analyzed in step (a), and classifying different bacteria based on the metabolite of the single living bacteriun obtained in step (a) to establish a comparison database for the bacterial identification.

10. A method for testing antibiotic susceptibility of a bacterium in a biological sample, comprising the method of any one of claims 1-9, and optionally further comprising a step of incubating the bacterium in the biological sample with the antibiotic.
